# EUROPÄISCHE PATENTSCHRIFT

(11) **EP 0 599 180 B1**
(45) Veröffentlichungstag und Bekanntmachung des Hinweises auf die Patenterteilung: **29.05.1996**
(21) Anmeldenummer: 93118476.6
(22) Anmeldetag: 16.11.1993
(51) Int. Cl.: C07C 209/48

(54) **Verfahren zur Herstellung von Diaminen**
Process for the preparation of diamines
Procédé pour la préparation de diamines

(30) Priorität: 26.11.1992 DE 4239782
(43) Veröffentlichungstag der Anmeldung: 01.06.1994
(73) Patentinhaber: BASF Aktiengesellschaft, D-67063 Ludwigshafen (DE)
(72) Erfinder: Witzel, Tom, Dr., D-6700 Ludwigshafen (DE); Fuchs, Eberhard, Dr., D-6700 Ludwigshafen (DE)

(56) Entgegenhaltungen:
- DE-A- 3 935 641
- GB-A- 1 157 638

## Beschreibung

Die vorliegende Erfindung betrifft ein Verfahren zur Herstellung von Diaminen aus Dinitrilen und sekundären Aminen an einem Palladium-Katalysator bei erhöhten Temperaturen und Drücken.

In Stud. Surf. Sci. Catal., 27 (1986) 105 bis 144 ist auf Seite 123 die Bildung tertiärer Amine aus sekundären Aminen und einem aliphatischen Nitril an einem Pd/C-Katalysator beschrieben, jedoch wird das Verfahren mit den Vorurteilen verworfen, daß die Reaktionsgeschwindigkeit mit steigender Startkonzentration des sekundären Amins sinkt und nur eine unbefriedigende Ausbeute erreicht wird.

Auch nach Ind. Tech. Bull., 11 (1970) 19 bis 24 läßt sich eine Synthese tertiärer Amine aus einem sekundären Amin und einem Nitril in technisch relevanter Ausbeute nicht erwarten. Zwar bildet sich das tertiäre Amin Tripentylamin aus Valeronitril an Pd/C mit 84 % Selektivität, der Umsatz beläuft sich aber nur auf unbefriedigende 28 %. Dagegen erhält man am gleichen Katalysator aus Valeronitril und Butylamin das sekundäre Butylpentylamin in 93 % bei 54 % Umsatz. Trotz vorhandenem sekundären Amin wird aber kein tertiäres Amin gebildet.

In Catalysis of Organic Reactions, Marcel Dekker, New York, Basel, 1992, Seite 103 wird die Verwendung von Katalysatorträgern wie Aluminiumoxid zur Herstellung von primären Aminen empfohlen, da die sauren Zentren des Trägers bereits gebildetes Amin abseits der aktiven Zentren adsorbieren und somit eine Verknüpfung verhindern.

Aus GB-A-1 157 637, GB-A-1 157 638 und GB-A-1 157 639 ist die Reaktion von 2-Methylglutarsäuredinitril mit Diethylamin in Gegenwart von Wasserstoff und Palladium auf Bariumsulfat oder bevorzugt Palladium auf Kohle zum 5-Diethylamino-2-methylvaleronitril bekannt. Trotz langer Reaktionszeit und einem Diethylaminüberschuß von 200 mol% wurde kein Tetraethylderivat gefunden, obwohl die Diethylaminogruppe nicht nur mit der Nitrilgruppen in 5-Position, sondern auch mit der in 1-Position reagiert (Verhältnis 4 zu 1).

Aus der DE-A-39 35 641 ist die Synthese eines sekundären Amins an einem Palladium-Katalysator beschrieben. Hier erfolgt die Reaktion von Dimethylaminopropionitril mit sich selbst unter Hydrierbedingungen zum Bis-(3-Dimethylaminopropyl)-amin. Die Bildung eines tertiären Amins wird lediglich an einem wesentlich aufwendiger herzustellenden Spinell als Trägermaterial in max. 58 % Ausbeute erreicht.

Aus der US-A-2 166 183 wird bei der Hydrierung von Dinitrilen mit 4, 5 oder 6 Kohlenstoffatomen vor der Bildung cyclischer sekundärer Amine gewarnt; im Falle von Adipodinitril also vor Hexamethylenimin.

Der vorliegenden Erfindung lag daher die Aufgabe zugrunde, den zuvor genannten Nachteilen abzuhelfen.

Demgemäß wurde ein neues und verbessertes Verfahren zur Herstellung von Diaminen der allgemeinen Formel I 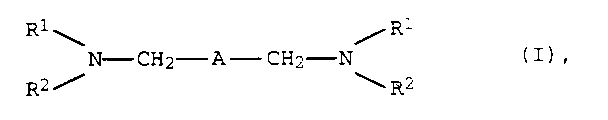
in der
- A: eine gegebenenfalls durch ein- bis fünffach durch C₁- bis C₄-Alkyl substituierte C₁- bis C₂₀-Alkylenkette,
- R¹, R²: C₁- bis C₂₀-Alkyl, C₁- bis C₂₀-Hydroxyalkyl, C₃- bis C₈-Cycloalkyl, C₄- bis C₂₀-Alkyl-cycloalkyl, C₄- bis C₂₀-Cycloalkyl-alkyl, Phenyl, C₇- bis C₂₀-Phenylalkyl, C₁- bis C₂₀-Alkoxyalkyl, C₇- bis C₂₀-Phenoxyalkyl oder gemeinsam eine gegebenenfalls durch ein- bis dreifach durch C₁- bis C₄-Alkyl substituierte gesättigte oder ungesättigte gegebenenfalls durch Sauerstoff oder Stickstoff unterbrochene C₂- bis C₆-Alkylenkette bedeuten,
durch Umsetzung von Dinitrilen der allgemeinen Formel II

NC―A―CN (II),

in der A die oben genannte Bedeutung hat, mit sekundären Aminen der allgemeinen Formel III 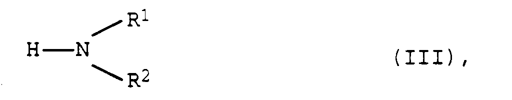
in der R¹ und R² die oben genannten Bedeutungen haben, mit Wasserstoff bei Temperaturen von 50 bis 200°C und Drücken von 5 bis 300 bar in Gegenwart eines Hydrierkatalysators gefunden, welches dadurch gekennzeichnet ist, daß man als Hydrierkatalysator Palladium-Katalysatoren auf oxidischen Trägern einsetzt.

Das erfindungsgemäße Verfahren läßt sich wie folgt durchführen:
Die Umsetzung der Dinitrile II mit den Diaminen III und mit Wasserstoff kann bei Temperaturen von 50 bis 200°C, bevorzugt 90 bis 170°C, besonders bevorzugt 120 bis 160°C und Drücken von 5 bis 300 bar, bevorzugt 50 bis 200 bar, besonders bevorzugt 70 bis 150 bar diskontinuierlich oder bevorzugt kontinuierlich in Druckapparaturen wie Autoklaven oder bevorzugt in einem Rohrreaktor an bestimmten Hydrierkatalysatoren durchgeführt werden.

Als Hydrierkatalysatoren eignen sich Palladium-Katalysatoren auf oxidischen Trägern. Als oxidische Träger eignen sich beispielsweise gamma-Al₂O₃, SiO₂, TiO₂, ZrO₂, mit Alkali- oder Erdalkalioxiden dotiertes Al₂O₃, SiO₂, TiO₂ oder ZrO₂.

Diese Palladium-Katalysatoren enthalten in der Regel 0,1 bis 10 Gew.-%, bevorzugt 0,3 bis 5 Gew.-% Palladium, besonders bevorzugt 0,5 bis 1 Gew.-%, bezogen auf das Gesamtgewicht des Katalysators.

Die Palladium-Katalysatoren sind allgemein bekannt oder können nach allgemein bekannten Verfahren, beispielsweise durch Tränken des Trägers mit Palladiumverbindungen wie PdCl₂ oder Pd(NO₃)₂, hergestellt werden.

Das Molverhältnis von sekundäre Amin III zum Nitril II beträgt in der Regel 2 : 1 bis 30 : 1, bevorzugt im 3 : 1 bis 10 : 1, besonders bevorzugt 4 : 1 bis 8 : 1.

Die im erfindungsgemäßen Verfahren entstehenden Diamine I lassen sich in an sich bekannter Weise, beispielsweise destillativ, reinigen.

Das Zwischenglied A und die Substituenten R¹ und R² in den Verbindungen I, II und III haben folgende Bedeutungen:
- A: - eine gegebenenfalls ein- bis fünffach durch C₁- bis C₄-Alkyl C₁- bis C₂₀-Alkylenkette wie -(CH₂)-, -(CH₂)₂-, -(CH₂)₃-, -(CH₂)₄-, -(CH₂)₅-, -(CH₂)₆-, -(CH₂)₇-, -(CH₂)₈-, -(CH₂)₉-, -(CH₂)₁₀-, -(CH₂)₁₁-, -(CH₂)₁₂-, -(CH₂)₁₃-, -(CH₂)₁₄-, -(CH₂)₁₅-, -(CH₂)₁₆-, -(CH₂)₁₇-, -(CH₂)₁₈-, -(CH₂)₁₉-, -(CH₂)₂₀-, -CH(CH₃)-CH₂-CH₂-, -CH₂-CH(CH₃)-CH₂-, -CH₂-C(CH₃)₂-CH₂-, bevorzugt -(CH₂)-, -(CH₂)₂-, -(CH₂)₃-, -(CH₂)₄-, -(CH₂)₆-CH₂-CH(CH₃)-CH₂-CH₂-, -CH₂-C(CH₂)-CH₂-CH₂ -CH₂-CH₂-CH(CN)-CH₂-CH₂-CH₂, besonders bevorzugt -(CH₂)₄-,
- R¹,R²: - C₁- bis C₂₀-Alkyl, bevorzugt C₁- bis C₁₂-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, n-Nonyl, iso-Nonyl, n-Decyl, iso-Decyl, n-Undecyl, iso-Undecyl, n-Dodecyl und iso-Dodecyl, besonders bevorzugt C₁- bis C₄-Alkyl wie Methyl, Ethyl, n-Propyl, iso-Propyl, n-Butyl, iso-Butyl, sec.-Butyl und tert.-Butyl,
- C₁- bis C₂₀-Hydroxyalkyl, bevorzugt C₂- bis C₈-Hydroxy-n-alkyl wie 2-Hydroxyethyl, 2-Hydroxy-n-propyl und 3-Hydroxy-n-propyl,
- C₃- bis C₈-Cycloalkyl wie Cyclopropyl, Cyclobutyl, Cyclopentyl, Cyclohexyl, Cycloheptyl und Cyclooctyl, bevorzugt Cyclopentyl, Cyclohexyl und Cyclooctyl, besonders bevorzugt Cyclopentyl und Cyclohexyl,
- C₄- bis C₂₀-Alkyl-cycloalkyl, bevorzugt C₄- bis C₁₂-Alkyl-cycloalkyl,
- C₄- bis C₂₀-Cycloalkyl-alkyl, bevorzugt C₄- bis C₁₂-Cycloalkyl-alkyl,
- Phenyl,
- C₇- bis C₂₀-Phenylalkyl, bevorzugt C₇- bis C₁₂-Phenylalkyl wie Benzyl, 1-Phenethyl, 2-Phenethyl, 1-Phenyl-propyl, 2-Phenyl-propyl, 3-Phenyl-propyl, 1-Phenyl-butyl, 2-Phenyl-butyl, 3-Phenyl-butyl und 4-Phenyl-butyl, besonders bevorzugt Benzyl, 1-Phenethyl und 2-Phenethyl,
- C₂- bis C₂₀-Alkoxyalkyl, bevorzugt C₂- bis C₈-Alkoxyalkyl wie Methoxymethyl, 2-Methoxy-ethyl, 2-Ethoxyethyl, 3-Methoxypropyl und 3-Ethoxypropyl,
- C₇- bis C₂₀-Phenoxyalkyl, bevorzugt C₇- bis C₁₂-Phenoxyalkyl wie 2-Phenoxyethyl, 2-Phenoxy-propyl, 3-Phenoxy-propyl, 2-Phenoxy-butyl, 3-Phenoxy-butyl und 4-Phenoxy-butyl, besonders bevorzugt 2-Phenoxyethyl,
- R¹,R²: - gemeinsam
- ein gegebenenfalls ein- bis dreifach durch C₁- bis C₄-Alkyl substituiertes C₂- bis C₆-Alkylen, wie -(CH₂)₂-, -(CH₂)₃-, -(CH₂)₄-, -(CH₂)₅-, -(CH₂)₆-, -CH(CH₃)-CH₂-CH₂- und -CH₂-CH(CH₃)-CH₂-,
- ein durch Sauerstoff und/oder Stickstoff unterbrochene und gegebenenfalls durch C₁- bis C₄-Alkyl substituiertes C₂- bis C₆-Alkylen wie -CH₂-CH₂-O-CH₂-CH₂, -CH₂-CH₂-NH-CH₂-CH₂-CH₂-CH₂-N(CH₃)-CH₂-CH₂-, CH₂-CH₂-N(CH₂CH₂)-CH₂-CH₂- und -CH₂-CH(CH₃)-O-CH(CH₃)-CH₂
Korksäuredinitril, Adipodinitril, Methylglutarsäuredinitril, Methylenglutarsäuredinitril, Glutarsäuredinitril, Bernsteinsäuredinitril, Malonsäuredinitril, 1,3,6-Tricyanohexan, bevorzugt Adipodinitril.

Dimethylamin, Diethylamin, Dipropylamin, Diisopropylamin, Di-n-butylamin, Diisobutylamin, Di-sec.-butylamin, Di-2-ethylhexylamin, Di-tridecylamin, Dicyclohexylamin, Ethyl-methylamin, Methyl-cylohexylamin, Ethyl-cyclohexylamin, Pyrrolidin, Piperidin, Piperazin, Morpholin, Diphenylamin, N-Methylanilin, N-Ethylanilin, Diethanolamin, Diisopropanolamin, Di-2-methoxyethylamin, Di-2-ethoxyethylamin, Methylethanolamin, Ethylethanolamin, Isopropylethanolamin, Hydroxyethylanilin, besonders bevorzugt Dimethylamin.

Besonders bevorzugt ist demnach N,N,N',N'-Tetramethylhexamethylen-diamin als Diamin I.

Diese tertiäre Amine sind Härter für Epoxidharze, Katalysatoren für Polyurethane, Zwischenprodukte zur Herstellung quartärer Ammoniumverbindungen, Weichmacher, Korrosionsinhibitoren, Textilhilfsmittel und Emulgatoren.

### Beispiele

- Katalysator A:: 4 % Pd auf Al₂O₃
- Katalysator B:: 0,5 % Pd auf Al₂O₃ mit 20 % CaO
- Katalysator C:: 1 % Pd auf Al₂O₃ mit 20 % MgO
- Katalysator D:: 0,5 % Pd; 5 % Pr auf Al₂O₃

### Beispiel 1

In einem Autoklaven wurde 10 g Adipodinitril mit 120 ml Dimethylamin (Molverhältnis 1 zu 20) an 1 g käuflichem Palladium auf Aluminiumoxid (Palladiumgehalt 5 %) als Katalysator über 10 Stunden bei 100°C und 200 bar hydriert. Man erhielt 12,7 g (80 %) Tetramethylhexamethylendiamin (Umsatz 100%; Selektivität 80 %).

### Beispiel 2

In einem Autoklaven wurde 100 g Adipodinitril mit 1270 ml Dimethylamin an 40 g Katalysator D 27 Stunden bei 150°C und 80 bar hydriert. Der Reaktionsaustrag enthielt 12,7 g (80 %) Tetramethylhexamethylendiamin, bei quantitativem Umsatz.

### Beispiel 3

Durch einen vertikalen Hydrierreaktor (Durchmesser: 16 mm; Füllhöhe : 600 mm; ölbeheizter Doppelmantel), der mit 28 g Katalysator A gefüllt war, wurde in Sumpffahrweise bei 80 bar und einer Temperatur von 150°C stündlich 8,1 ml Adipodinitril und 43 ml flüssiges Dimethylamin (Molverhältnis 1 zu 5) gepumpt. Gleichzeitig leitete man 10 Nl/h Wasserstoff von unten nach oben durch den Reaktor. Nach Entspannen auf Normaldruck wurde überschüssiges Dimethylamin abdestilliert und der Hydrieraustrag durch quantitative Gaschromatographie analysiert. Man erhielt stündlich 11 g (86 %) Tetramethylhexamethylendiamin.

### Beispiel 4

In der in Beispiel 3 beschriebenen Apparatur erhielt man an 43 g Katalysator B bei 80 bar und 150°C aus stündlich 11,3 ml Adipodinitril und 34 ml Dimethylamin (Molverhältnis 1 zu 5) 16,7 g (93 %) Tetramethylhexamethylendiamin.

### Beispiel 5

In der in Beispiel 3 beschriebenen Apparatur erhielt man an 40 g Katalysator C bei 80 bar und 150°C aus stündlich 10,5 ml Adipodinitril und 32 ml Dimethylamin (Molverhältnis 1 zu 5) 15,7 g (94 %) Tetramethylhexamethylendiamin.

### Beispiel 6

In der in Beispiel 3 beschriebenen Apparatur erhielt man an 39 g Katalysator D bei 80 bar und 155°C aus stündlich 8,2 ml Adipodinitril und 25 ml Dimethylamin (Molverhältnis 1 zu 5) 12,1 g (93 %) Tetramethylhexamethylendiamin.

## Patentansprüche

1. Verfahren zur Herstellung von Diaminen der allgemeinen Formel I 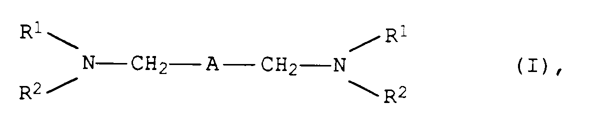 in der
A eine gegebenenfalls durch ein- bis fünffach durch C₁- bis C₄-Alkyl substituierte C₁- bis C₂₀-Alkylenkette,
R¹, R² C₁- bis C₂₀-Alkyl, C₁- bis C₂₀-Hydroxyalkyl, C₃- bis C₈-Cycloalkyl, C₄- bis C₂₀-Alkyl-cycloalkyl, C₄- bis C₂₀-Cycloalkyl-alkyl, Phenyl, C₇- bis C₂₀-Phenylalkyl, C₁- bis C₂₀-Alkoxyalkyl, C₇- bis C₂₀-Phenoxyalkyl oder gemeinsam eine gegebenenfalls durch ein- bis dreifach durch C₁- bis C₄-Alkyl substituierte gesättigte oder ungesättigte gegebenenfalls durch Sauerstoff oder Stickstoff unterbrochene C₂- bis C₆-Alkylenkette bedeuten,
durch Umsetzung von Dinitrilen der allgemeinen Formel II
NC―A―CN (II),
in der A die oben genannte Bedeutung hat, mit sekundären Aminen der allgemeinen Formel III 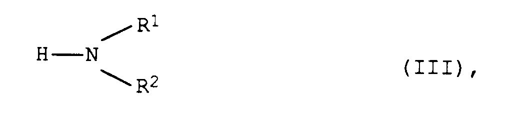 in der R¹ und R² die oben genannten Bedeutungen haben, mit Wasserstoff bei Temperaturen von 50 bis 200°C und Drücken von 5 bis 300 bar in Gegenwart eines Hydrierkatalysators, dadurch gekennzeichnet, daß man als Hydrierkatalysator Palladium-Katalysatoren auf oxidischen Trägern einsetzt.

2. Verfahren zur Herstellung von Diaminen der Formel I nach Anspruch 1, dadurch gekennzeichnet, daß man als Hydrierkatalysator Palladium-Katalysatoren auf gamma-Al₂O₃, SiO₂, TiO₂ oder ZrO₂ oder mit Alkali- oder Erdalkalioxiden dotiertes Al₂O₃, SiO₂, TiO₂ oder ZrO₂ als oxidischen Trägern einsetzt.

## Claims

1. A process for the preparation of diamines of the formula I 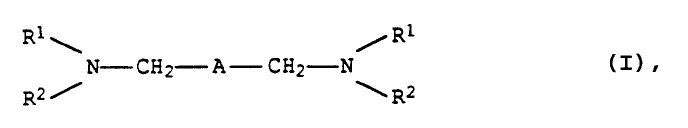 where
A is a C₁-to C₂₀-alkylene chain which is unsubstituted or monosubstituted to pentasubstituted by C₁- to C₄-alkyl,
R¹ and R² are C₁- to C₂₀-alkyl, C₁- to C₂₀-hydroxyalkyl, C₃- to C₈-cycloalkyl, C₄- to C₂₀-alkylcycloalkyl, C₄-to C₂₀-cycloalkyl, phenyl, C₇- to C₂₀-phenylalkyl, C₁- to C₂₀-alkoxyalkyl, C₇- to C₂₀-phenoxyalkyl or together are a saturated or unsaturated C₂- to C₆-alkylene chain which is unsubstituted or monosubstituted to trisubstituted by C₁- to C₄-alkyl and may be interrupted by oxygen or nitrogen,
by reacting dinitriles of the formula II
NC-A-CN (II),
where A is as defined above, with secondary amines of the formula III 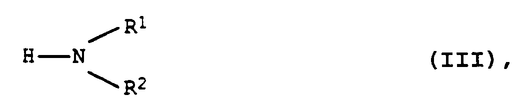 where R¹ and R² are as defined above, with hydrogen at from 50 to 200°C and at from 5 to 300 bar in the presence of a hydrogenation catalyst, wherein the hydrogenation catalyst is a palladium catalyst on an oxidic support.

2. A process for the preparation of diamines of the formula I as claimed in claim 1, wherein the hydrogenation catalyst is a palladium catalyst on gamma-Al₂O₃, SiO₂, TiO₂ or ZrO₂ or Al₂O₃, SiO₂, TiO₂ or ZrO₂ doped with alkali or alkaline earth metal oxides as oxidic support.

## Revendications

1. Procédé de préparation de diamines de la formule I 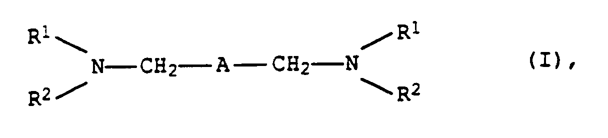 dans laquelle
A représente une chaîne alkylène en C₁ à C₂₀ éventuellement une à cinq fois substituée par des radicaux alkyle en C₁ à C₄,
R¹, R² représentent chacun un radical alkyle en C₁ à C₂₀, hydroxyalkyle en C₁ à C₂₀, cycloalkyle en C₃ à C₈, alkylcycloalkyle en C₄ à C₂₀, cycloalkylalkyle en C₄ à C₂₀, phényle, phénylalkyle en C₇ à C₂₀, alcoxyalkyle en C₁ à C₂₀, phénoxyalkyle en C₇ à C₂₀, ou bien R¹ et R² forment, en commun, une chaîne alkylène en C₂ à C₆, saturée ou insaturée, éventuellement interrompue par de l'oxygène ou de l' azote, éventuellement une à trois fois substituée par des radicaux alkyle en C₁ à C₄,
par la réaction de dinitriles de la formule II
NC―A―CN (II),
dans laquelle A possède les significations qui lui ont été attribuées ci-dessus, avec des amines secondaires de la formule générale III 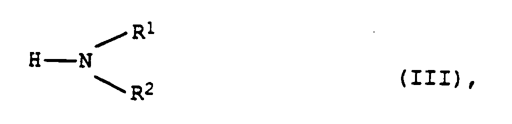 dans laquelle R¹ et R² ont les significations qui leur ont été attribuées ci-dessus, avec de l'hydrogène, à des températures de 50 à 200°C et sous des pressions de 5 à 300 bars, en présence d'un catalyseur d'hydrogénation, caractérisé en ce que l'on utilise, à titre de catalyseur d'hydrogénation, des catalyseurs au palladium sur des supports oxydiques.

2. Procédé de préparation de diamines de la formule générale I suivant la revendication 1, caractérisé en ce que, à titre de catalyseur d'hydrogénation, on utilise des catalyseurs au palladium sur gamma-Al₂O₃, SiO₂, TiO₂ ou ZrO₂, ou sur Al₂O₃, SiO₂, TiO₂ ou ZrO₂, dopé avec des oxydes de métaux alcalins ou alcalino-terreux, à titre de supports oxydiques.
